# FASCICULE DE BREVET EUROPEEN

(11) **EP 1 904 063 B1**
(45) Date de publication et mention de la délivrance du brevet: **20.02.2019**
(21) Numéro de dépôt: 06778813.3
(22) Date de dépôt: 07.07.2006
(51) Int. Cl.: A61K 31/426, A61K 31/427, A61P 25/14

(54) **DERIVES DE THIAZOLES POUR TRAITER LES DYSKINESIES PROVOQUEES PAR UN TRAITEMENT CHIMIQUE**
THIAZOL-DERIVATE ZUR BEHANDLUNG VON DYSKINESIEN, DIE DURCH EINE CHEMISCHE BEHANDLUNG AUSGELÖST WURDEN
THIAZOLE DERIVATIVES FOR THE TREATMENT OF DYSKINESIAS CAUSED BY A CHEMICAL TREATMENT

(30) Priorité: 08.07.2005 FR 0507303
(43) Date de publication de la demande: 02.04.2008
(73) Titulaire: IPSEN PHARMA, 92100 Boulogne-Billancourt (FR)
(72) Inventeur: CHABRIER DE LASSAUNIERE, Pierre-Etienne, F-75016 Paris (FR); AUGUET, Michel, F-91120 Palaiseau (FR); SPINNEWYN, Brigitte, F-91440 Bures Sur Yvette (FR)
(74) Mandataire: Audonnet, Nathalie
(86) Numéro de dépôt international: PCT/FR2006/001638
(87) Numéro de publication internationale: WO 2007/006942

(56) Documents cités:
- WO-A-02/083656
- WO-A2-01/26656
- LEES A: "Alternatives to levodopa in the initial treatment of early Parkinson's disease" DRUGS AND AGING 2005 NEW ZEALAND, vol. 22, no. 9, février 2005 (2005-02), pages 731-740, XP009064944 ISSN: 1170-229X
- BIRKMAYER W ET AL: "THE POTENTIATION OF THE ANTI AKINETIC EFFECT AFTER L-DOPA TREATMENT BY AN INHIBITOR OF MAO-B, DEPRENIL*" JOURNAL OF NEURAL TRANSMISSION, SPRINGER VERLAG, VIENNA, AT, vol. 36, 1975, pages 303-326, XP000575120 ISSN: 0300-9564
- HEINTZ R ET AL: "PARGYLINE REDUCES-PREVENTS NEUROLEPTIC-INDUCED ACUTE DYSTONIA IN MONKEYS" PSYCHOPHARMACOLOGY, vol. 93, no. 2, 1987, pages 207-213, XP009064996 ISSN: 0033-3158
- KOE B K: "MONO AMINE OXIDASE INHIBITORS ANTAGONIZE THE ACCELERATION OF BRAIN DOPAMINE SYNTHESIS INDUCED BY NEUROLEPTIC DRUGS IN-VIVO IMPLICATIONS FOR THE TREATMENT OF TARDIVE DYS KINESIA" EXPERIENTIA (BASEL), vol. 31, no. 6, 1975, pages 669-671, XP009065015 ISSN: 0014-4754
- TREBINI F ET AL: "CLINICAL EVALUATION OF SELEGILINE L DEPRENYL IN THE LONG-TERM L DOPA TREATMENT SYNDROME" ACTA NEUROLOGICA (NAPLES), vol. 40, no. 5, 1985, pages 432-439, XP009065047 ISSN: 0001-6276

## Description

La présente invention a pour objet des dérivés de thiazoles de formule générale (I) décrite ci-dessous pour préparer un médicament destiné à traiter ou à prévenir les dyskinésies provoquées par un traitement chimique.

D'une manière générale les dyskinésies se caractérisent par des troubles involontaires ou anormaux du mouvement.

Parmi les dyskinésies, on distingue celles provoquées par un traitement chimique. Dans ce cas on parle, au sens de la présente invention, de dyskinésies induites ou de dyskinésies tardives, ces 2 formes de dyskinésies étant des dyskinésies provoquées par un traitement chimique.

Les dyskinésies induites : elles sont induites par un traitement chimique, comme par exemple chez les patients traités par la L-Dopa qui est un précurseur de la dopamine. Les patients traités par la L-Dopa sont généralement des parkinsoniens, c'est-à-dire des personnes atteintes de la maladie de Parkinson qui présentent comme caractéristique principale de la maladie un déficit pathologique de Dopamine. Le traitement par dopathérapie est absolument nécessaire pour ces malades afin de restaurer un niveau de dopamine endogène suffisant. Il provoque cependant chez un nombre important de patients des effets secondaires sévères se manifestant par des dyskinésies dites induites, et ceci après généralement plusieurs années de traitement à la L-Dopa. Cette forme de dyskinésie qui est un effet iatrogène indésirable grave de la L-Dopa se traduit notamment par l'émergence progressive de mouvements involontaires, une rigidité musculaire et des troubles de la démarche. L'émergence de ce type de dyskinésie n'intervient jamais chez des patients parkinsonien « de novo », n'ayant jamais reçu auparavant un traitement à la L-Dopa. Malheureusenent, il n'existe pas de traitement satisfaisant à l'heure actuelle des dyskinésies induites par la Dopathérapie. Le clinicien prenant en charge un parkinsonien atteint de dyskinésie induite par la L-Dopa est alors contraint de diminuer la dose quotidienne de L-Dopa pour réduire la sévérité des mouvements anormaux (dyskinésie), ce qui s'accompagne alors par une aggravation des phénomènes de blocage due à la maladie de Parkinson. Le clinicien et le patient doivent alors choisir entre 2 maux : blocages ou dyskinésie.

Il n'existe pas de traitements médicamenteux satisfaisants à ce jour des dyskinésies induites par la L-Dopa (quelques composés ont été proposés, comme par exemple la yohimbine, l'idazoxan ou l'amantadine). Seule la chirurgie interventionnelle par stimulation du globus pallidus constitue une stratégie thérapeutique qui pourrait être efficace dans les cas les plus sévères, mais qui n'est évidemment pas adaptable à la majorité des patients.

Les dyskinésies tardives : elles apparaissent tardivement chez des patients traités de longue date par certains médicaments en particulier par des neuroleptiques. Il s'agit à titre d'exemple des patients atteints de schizophrénie traités par les neurolopetiques typiques (comme par exemple les agonistes des récepteurs dopaminergiques D2) comme l'haloperidol, ou par des agonistes dits atypiques (ligands des récepteurs dopaminergiques D3 ou D4) comme la clozapine ou l'olanzepine. Ces traitements provoquent comme effets secondaires des dyskinésies dites tardives. Il s'agit notamment de mouvements anormaux, involontaires, aux niveaux faciaux, buccaux, linguaux, masticateurs et de mouvements, diffus, de balancements rythmés du tronc, de dandinements, et de piétinements ;

Or les traitements thérapeutiques connus à ce jour ne sont pas satisfaisants.

Afin de répondre aux besoins des patients, il est devenu nécessaire de trouver un nouveau moyen pour traiter ces dyskinésies provoquées par un traitement chimique qui sont très invalidantes pour les patients.

Aussi le problème que se propose de résoudre l'invention est de fournir un nouveau composé adapté pour traiter les dyskinésies provoquées par un traitement chimique.

De manière inattendue, la Demanderesse a mis en évidence qu'il est possible d'utiliser des dérivés de thiazoles de formule générale (I) décrite ci-dessous pour préparer un médicament destiné à traiter ou à prévenir les dyskinésies provoquées par un traitement chimique.

De manière encore plus inattendue, la Demanderesse a mis en évidence que l'association des dérivés de thiazoles de formule générale (I) décrite ci-dessous et d'au moins un composé choisi parmi les produits agissant sur le système dopaminergique ou les neuroleptiques permet de traiter ou de prévenir les dyskinésies provoquées par un traitement chimique.

Enfin l'utilisation et l'association selon l'invention ont pour avantage de pouvoir être mises en oeuvre chez les patients et les animaux atteints de troubles du mouvement.

L'association selon l'invention a pour avantage d'éviter les effets secondaires provoqués par un traitement thérapeutique à long terme.

D'autres avantages et caractéristiques de l'invention apparaîtront clairement à la lecture de la description et des exemples donnés à titre purement illustratifs et non limitatifs qui vont suivre.

Un premier objet de l'invention est l'utilisation d'un composé de formule générale **(I)** 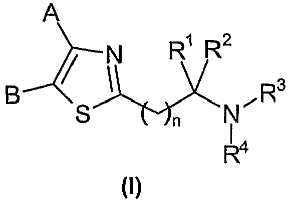 sous forme de mélange racémique, d'énantiomère ou de toute combinaison de ces formes, dans laquelle :
A représente un radical **(A1)** 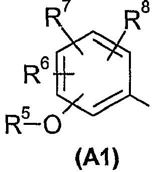
dans lequel R⁵ représente un atome d'hydrogène, R⁶, R⁷, R⁸ représentent indépendamment un atome d'hydrogène ou un radical alkyle;
B représente un atome d'hydrogène ou un radical alkyle ;
n est égal à 0;
R¹ représente un atome d'hydrogène et R² représente un atome d'hydrogène ou un radical alkyle ou cycloalkyle ;
R³ et R⁴ représentent indépendamment un atome d'hydrogène ou un radical alkyle ;
ou d'un sel de formule générale (I) définie ci-dessus pour préparer un médicament destiné à traiter ou à prévenir les dyskinésies provoquées par un traitement chimique.

Au sens de la présente invention, on entend par radical alkyle ou alkoxy, lorsqu'il n'est pas donné plus de précisions, un radical alkyle ou alkoxy linéaire ou ramifié et comptant de 1 à 6 atomes de carbone. Par radical cycloalkyle, lorsqu'il n'est pas donné plus de précisions, on entend un radical cycloalkyle comptant de 3 à 7 atomes de carbone.

De préférence, par alkyle linéaire ou ramifié ayant de 1 à 6 atomes de carbone, on entend en particulier les radicaux méthyle, éthyle, propyle, isopropyle, butyle, isobutyle, sec-butyle et tert-butyle, pentyle, néopentyle, isopentyle, hexyle, isohexyle.

Par sel pharmaceutiquement acceptable, on entend notamment au sens de l'invention des sels d'addition d'acides inorganiques tels que chlorhydrate, bromhydrate, iodhydrate, sulfate, phosphate, diphosphate et nitrate ou d'acides organiques tels que acétate, maléate, fumarate, tartrate, succinate, citrate, lactate, méthanesulfonate, p-toluènesulfonate, pamoate et stéarate. Entrent également dans le champ de la présente invention, lorsqu'ils sont utilisables, les sels formés à partir de bases telles que l'hydroxyde de sodium ou de potassium. Pour d'autres exemples de sels pharmaceutiquement acceptables, on peut se référer à "Salt selection for basic drugs", Int. J. Pharm. (1986), 33, 201-217.

Par ailleurs, certains des composés de formule générale (I) peuvent se présenter sous la forme d'énantiomères. La présente invention inclut les deux formes énantiomères et toutes combinaisons de ces formes, y compris les mélanges racémiques "R, S". Dans un souci de simplicité, lorsque aucune configuration spécifique n'est indiquée dans les formules de structure, il faut comprendre que les deux formes énantiomères et leurs mélanges sont représentés.

De préférence, les composés selon l'invention seront tels qu'il s'agit des composés suivants :
- 4-[2-(aminométhyl)-1,3-thiazol-4-yl]-2,6-di(*tert*-butyl)phénol;
- 4-{2-[(1R)-1-aminoethyl]-1,3-thiazol-4-yl}-2,6-di-tert-butylphenol ;
- 4-{2-[(1S)-1-aminoethyl]-1,3-thiazol-4-yl}-2,6-di-tert-butylphenol;

De préférence, l'invention a pour but l'utilisation d'un composé de formule générale **(I)** ou un sel de formule générale **(I)** définie ci-dessus pour préparer un médicament destiné à traiter ou à prévenir les dyskinésies provoquées par un traitement chimique comme par exemple la dyskinésie induite ou la dyskinésie tardive.

Préférentiellement, l'invention a pour objet l'utilisation d'un composé de formule générale **(I)** ou un sel de formule générale **(I)** définie ci-dessus pour préparer un médicament destiné à traiter ou à prévenir les dyskinésies induites comme par exemple les dyskinésies induites par la lévodopa (L-Dopa), par l'assocation lévodopa-benzerazide ou l'association levodopa-carbodopa, par le pergolide, le quinpirole, la carbergoline, la benzotropine, le trihexylphénidyl, le ropinorole, le pramipexole et tout autres dérivés dopaminergiques se substituant à la dopamine.

Préférentiellement, l'invention a pour objet l'utilisation d'un composé de formule générale **(I)** ou un sel de formule générale **(I)** définie ci-dessus pour préparer un médicament destiné à traiter ou à prévenir les dyskinésies tardives comme par exemple les dyskinésies tardives suite à un traitement par des dérivés de phénothiazine, des dérivés de butyrophénones, l'haloperidol, la risperidone, les dérivés de tetrabenazine, la clozapine, l'olanzapine, la fluoxétine, la buspirone.

L'utilisation selon invention ne concerne pas le traitement des dyskinésies non-provoquées par un traitement chimique.

Par dyskinésies non-provoquées par un traitement chimique, on entend au sens de l'invention les mouvements anormaux pathologiques apparaissant comme signe clinique d'une maladie neurodégénérative.

L'utilisation selon invention ne concerne pas le traitement des mouvements anormaux pathologiques de la maladie de Huntington.

Par dyskinésies provoquées par un traitement chimique, on entend au sens de l'invention les dyskinésies qui se déclarent après administration d'une substance chimique, qui sera à l'origine de cette dyskinésie (effet iatrogène). Dans ce cas la dyskinésie provoquée par ledit traitement chimique sera soit une pathologie principale, soit un effet secondaire de ce traitement chimique. Il est à noter que la dyskinésie provoquée suite à un traitement chimique, peut se déclarer pendant l'administration du composé ou après l'arrêt du traitement. On pourra distinguer les dyskinésies de milieu de dose, ou encore de début ou de fin de dose.

Un second objet de l'invention est une association d'un composé de formule générale **(I)** ou un sel de formule générale **(I)** définie ci-dessus avec un ou plusieurs autres composés chimiques, ayant ou non un effet thérapeutique, en particulier avec un composé ayant un effet psychotrope.

De préférence, l'association selon l'invention est un composé de formule générale **(I)** ou un sel de formule générale **(I)** définie ci-dessus et au moins un composé choisi parmi les agonistes dopaminergiques, les inhibiteurs de MAO, les inhibiteurs de catécholamine O-methyltransférase ou les neuroleptiques.

Parmi les agonistes dopaminergiques, on peut citer entre autre le pergolide, la bromocriptine ou la carbergoline, le ropinirole ou le pramipexole.

Plus préférentiellement, le composé selon l'invention de formule générale **(I)** définie ci-dessus ou son sel peut être utilisé en association avec la methyldopa ou encore la L-Dopa.

De manière encore plus préférentielle, l'association selon l'invention est un composé de formule générale **(I)** définie ci-dessus ou son sel et de la L-Dopa.

De manière particulière, l'invention concerne l'association du 4-[2-(aminométhyl)-1,3-thiazol-4-yll-2,6-di(*tert*-butyl)phénol ou son sel et la L-Dopa.

De manière particulière, l'invention concerne l'association du 4-{2-[(1R)-1-aminoethyl]-1,3-thiazol-4-yl}-2,6-di-tert-butylphenol ou son sel et la L-Dopa.

De manière particulière, l'invention concerne l'association du 4-{2-[(1S)-1-aminoethyl]-1,3-thiazol-4-yl}-2,6-di-tert-butylphenol ou son sel et la L-Dopa.

L'association selon l'invention est de préférence une préparation combinée pour une utilisation simultanée, séparée ou étalée dans le temps pour traiter ou prévenir les dyskinésies, en particulier les dyskinésies provoquées par un traitement chimique, en particulier les dyskinésies induites ou tardives.

Selon une variante de l'utilisation ou de l'association selon l'invention, il est possible d'utiliser ou d'associer à l'invention un inhibiteur de la décarboxylase tel que le bensérazide ou la carbidopa.

Le composé de formule générale **(I)** ou son sel utilisé selon l'invention ou l'association selon l'invention peuvent être sous forme solide, par exemple des poudres, des granules, des comprimés, des gélules, des liposomes ou des suppositoires. Les supports solides appropriés peuvent être, par exemple, le phosphate de calcium, le stéarate de magnésium, le talc, les sucres, le lactose, la dextrine, l'amidon, la gélatine, la cellulose, la cellulose de méthyle, la cellulose carboxyméthyle de sodium, la polyvinylpyrrolidine et la cire.

Le composé de formule générale **(I)** ou son sel utilisé selon l'invention ou l'association selon l'invention peuvent aussi se présenter sous forme liquide, par exemple, des solutions, des émulsions, des suspensions ou des sirops. Les supports liquides appropriés peuvent être, par exemple, l'eau, les solvants organiques tels que le glycérol ou les glycols, de même que leurs mélanges, dans des proportions variées, dans l'eau.

L'administration d'un composé de formule générale **(I)** ou son sel utilisé selon l'invention ou l'association selon l'invention pourra se faire par exemple par voie topique, orale, parentérale, par injection intramusculaire ou sous-cutanée. La dose d'administration envisagée pourra être comprise entre 0,01 mg et 10 g suivant le type de composé actif utilisé.

Les exemples suivants illustrent l'invention sans en limiter la portée.

La figure 1 représente l'effet du traitement avec les composés selon l'invention sur des rats atteints de dyskinésie induite.

La figure 2 représente l'effet du traitement avec les composés selon l'invention sur des rats atteints de dyskinésie tardive.

### EXEMPLES

Les composés suivants :
- Composé 1 : chlorhydrate ou dichlorhydrate de 4-[2-(aminométhyl)-1,3-thiazol-4-yl]-2,6-di(*tert*-butyl)phénol ;
- Composé 2 : chlorhydrate de 4-{2-[(1R)-1-aminoethyl]-1,3-thiazol-4-yl}-2,6-di-tert-butylphenol.
ont été soumis au test d'étude des dyskinésies induites par la L-DOPA chez le rat et au test d'étude des dyskinésies tardives après traitement de manière chronique par un neuroleptique chez le rat.

La synthèse de ces composés est décrite dans la demande WO 01/26656.

### EXEMPLE 1 : Etude des dyskinésies induites par la L-DOPA

### Principe du test in vivo (modèle expérimental de rats dyskinétiques) :

Un modèle animal est réalisé où la dyskinésie est induite chez des animaux traités avec de la L-Dopa (3-(3,4-dihydroxyphényl)-L-alanine). Tout d'abord afin de provoquer une diminution du taux de dopamine endogène au niveau du striatum, des rats sont lésés avec de la 6-hydroxydopamine (6-OHDA). Suite à cette injection de 6-OHDA, ces animaux deviennent sensibles aux effets secondaires de la L-dopa. En effet pour palier cette déficience en dopamine endogène, les rats reçoivent un traitement à base de L-Dopa. Cependant le traitement à la L-Dopa provoque une dyskinésie chez le rat suite à de trop grandes quantités de L-Dopa exogène qui arrivent au cerveau et surtout à cause de la lésion des neurones dopaminergiques qui ne régulent plus le taux de dopamine endogène. La dyskinésie ainsi induite est évaluée par un test statistique comportemental.

*Remarque* : pour améliorer le traitement à base de L-Dopa, celle-ci est administrée en association avec un inhibiteur de décarboxylase tel que le bensérazide. En effet la L-Dopa traverse difficilement la barrière hématoencéphalique et donc elle peut être décarboxylée au niveau périphérique.

### Mesure des dyskinésies :

### 1/ Sélection de rats:

L'étude a été réalisée sur des jeunes rats mâles. Les rats sont anesthésiés (chloral 400 mg/kg). Ensuite ils reçoivent 2 injections stéréotaxiques de 6 µg de 6-hydroxydopamine dans le striatum gauche en utilisant une microseringue Hamilton de 10 µl. Les rats reçoivent in fine 12 µg de 6-OHDA dissoute dans 4 µl de L-ascorbate à 0,02 %. Quatre semaines plus tard la lésion de la substance noire est constituée chez les rats. Pour quantifier cette lésion, on utilise une mesure indirecte : les rotations induites par l'apomorphine (test de substance). Les animaux reçoivent une injection sous-cutanée de 0,5 mg/kg d'apomorphine et les rotations droites et gauches des animaux sont mesurées pendant 30 minutes. Un score net d'asymétrie rotationnelle est alors déterminé. Ce score est exprimé comme le nombre de tours complets dans la cage de test / minute dans la direction contralatérale de la lésion.

Les rats qui présentent une moyenne individuelle supérieure à 4 tours complets / minute dans la direction contralatérale de la lésion sont sélectionnés pour réaliser le test in vivo. Ce score net d'asymétrie rotationnelle correspond à des animaux dont la substance noire est fortement lésée et qui présentent au moins 90 % de déplétion en dopamine dans le striatum (Lee et al.; 1996; Lundblad et al., 2002; Winkler et al., 1996).

### 2/ Induction de la dyskinésie chez les rats sélectionnés :

Six semaines après les lésions à la 6-hydroxydopamine (6-OHDA), les rats sélectionnés reçoivent chaque jour une injection intra péritonéale de methylester de la L-Dopa à la dose de 20 mg/kg et de bensérazide à la dose de 15 mg/kg. Les injections ont lieu de la semaine 6 à la semaine 9. A partir de la semaine 10, les rats reçoivent une dose plus concentrée : chaque jour ils reçoivent une injection intra péritonéale de methylester de la L-Dopa à la dose de 25 mg/kg et de bensérazide à la dose de 15 mg/kg. A partir de la semaine 11, les rats reçoivent deux injections quotidiennes de méthylester de la L-Dopa à la dose de 15 mg/kg et de bensérazide à la dose de 15 mg/kg. Pendant la semaine 11, les rats sont évalués 3 fois par semaine pour leurs taux de mouvements anormaux et involontaires afin de déterminer si les doses administrées sont suffisantes pour provoquer une dyskinésie chez ces animaux. Les rats sont observés individuellement à partir de 30 minutes et jusqu'à 180 minutes après injection de la L-Dopa ; ils sont observés toutes les 30 minutes. Les mouvements qui sont observés à ce moment là sont considérés comme des mouvements dyskinétiques lorsqu'ils remplissent les critères suivants :
- les mouvements sont induits par la L-Dopa ;
- les mouvements affectent le coté du corps dans la direction contralatérale de la lésion ;
- les mouvements sont répétitifs, involontaires et non attribuables à un comportement normal.

Les mouvements anormaux et involontaires sont classés en 4 sous-types :
- dyskinésie locomotive : augmentation de la locomotion du coté contralatéral à la lésion ;
- dystonie axiale : déviation de la posture du cou et de la partie supérieure du corps ;
- dyskinésie oromandibulaire : mouvements des mâchoires stéréotypés et protrusion de la langue ;
- dyskinésie des membres antérieurs : secousses répétitives et rythmées des membres antérieurs accompagnées d'une posture dystonique et / ou de mouvements vifs de saisie des pattes.
Pour chacun de ces sous-types, les rats sont notés sur une échelle de 0 à 4, sachant que :
- 0 = aucun mouvement,
- 1 = mouvements occasionnels,
- 2 = mouvements fréquents,
- 3 = mouvements continus mais interrompus par une distraction sensorielle,
- 4 = mouvements continus, aigus, non interrompus par une distraction sensorielle.

Les composés selon l'invention sont testés afin de déterminer leur capacité à moduler les effets de la L-Dopa. Dans ce but, les rats qui présentent un taux de mouvements anormaux et involontaires suffisant (taux supérieur à 2 pour un mouvement observé et taux total supérieur à 8 pour les 4 mouvements observés) sont sélectionnés et continuent de recevoir 2 à 4 injections / semaine de L-Dopa. Après 12 à 14 semaines, le traitement oral avec les composés selon l'invention à tester démarre et le taux de mouvements anormaux et involontaires est évalué.

Pour chaque animal recevant un composé à tester, un autre animal reçoit le solvant du composé à tester, en général de l'eau.

### Résultats :

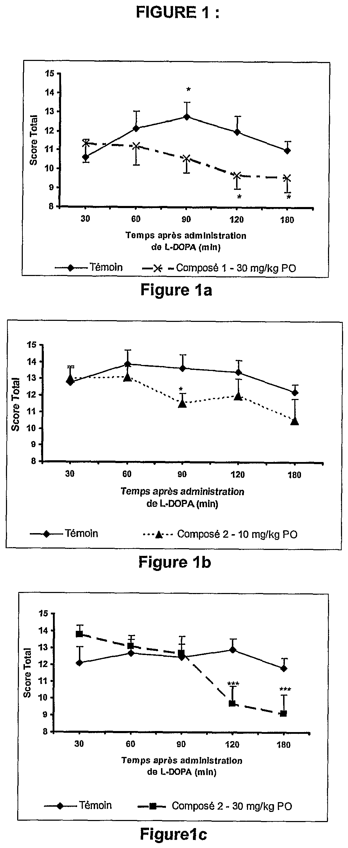

Le taux de mouvements anormaux et involontaires est calculé de manière statistique pour chaque groupe de rats. Les résultats sont présentés sur la figure 1 pour les composés 1 et 2.

Le composé 1 selon l'invention est administré oralement à la dose de 30 mg/kg à des rats dyskinétiques selon le protocole de l'exemple 1 décrit ci-dessus. Les résultats présentés ont été obtenus avec 9 animaux testés.

Le composé 2 selon l'invention est administré oralement à la dose de 10 et 30 mg/kg à des rats dyskinétiques selon le protocole de l'exemple 1 décrit ci-dessus. Les résultats présentés ont été obtenus avec 9 animaux testés.

Le score obtenu permet d'évaluer la dyskinésie du rat. Plus la valeur numérique du score est élevée, plus l'animal est dyskinétique avec un maximum de 16 points.
- composé 1 : à la dose de 30 mg/kg, le score diminue de 11,3 à 9,6 après 180 minutes de traitement.
   Au temps 120 minutes après l'administration de L-Dopa, les rats traités avec le composé 1 ont un score de 10 points alors que le témoin (eau) présente un score de 12 points ;
- Composé 2 : à la dose de 30 mg/kg, le score diminue de 13,8 à 9 points et à la dose de 10 mg/kg, le score diminue de 13 à 10,6 points après 180 minutes de traitement.
   Au temps 120 minutes après l'administration de L-Dopa, les rats traités avec le composé 2 (30 mg/kg) ont un score de 9,7 points alors que le témoin (eau) présente un score de 12,9.

Les résultats indiquent une efficacité dose dépendante du traitement avec les composés 1 et 2 sur les rats atteints de dyskinésie induite. Cette efficacité est significative statistiquement. Les composés 1 et 2 ont bien un effet anti-dyskinétique.

### EXEMPLE 2 : Etude des dyskinésies tardives chez le rat traité de manière chronique par un neuroleptique

### Principe du test in vivo (modèle expérimental de rats) :

La dyskinésie tardive est une complication suite à un traitement prolongé avec par exemple des neuroleptiques. Elle se caractérise par des mouvements répétitifs, involontaires de la bouche, de la face et de la langue. Un traitement chronique avec des neuroleptiques peut conduire à une dyskinésie oromandibulaire, en particulier peut conduire à des mouvements de mastication et protrusion de la langue chez le rat. Le principe du test est de quantifier les mouvements anormaux des animaux dans une cage de test pendant une fenêtre de temps déterminée (5 minutes).

### Induction de la dyskinésie orofacial(e) chez des rats:

Un neuroleptique, l'halopéridol (provenant de chez Sigma®), est administré de manière chronique à des rats en utilisant une pompe osmotique de marque Alzet®. Cette pompe osmotique (référence 2ML4 Alzet®) est remplie avec une solution d'halopéridol à 10 mg/ml. Cette pompe est implantée en sous-cutané à des rats de poids compris entre 150 g et 160 g. Après anesthésie des rats avec de l'isoflurane, une petite incision est réalisée dans la peau au niveau du scrapulae pour permettre d'insérer la pompe. Puis l'incision est suturée. La pompe permet d'administrer en continu de l'halopéridol aux rats à la dose de 2 mg/kg/jour pendant 28 jours.

### Mesure de la dyskinésie chez des rats :

Entre 21 à 28 jours après la pose de la pompe, 12 rats sont choisis au hasard pour le test. 6 d'entres eux sont traités avec de l'eau (témoin), et les 6 autres rats reçoivent les composés selon l'invention.
Avant le traitement (t=0) avec les composés à tester, les rats sont placés individuellement dans des cages de test en plastique. Après un temps d'adaptation dans la cage de 2 minutes, le nombre de protrusion de la langue et de mouvements de mastication est compté pendant 5 minutes. Puis le traitement (composés 1 et 2 à tester) est administré oralement à la dose de 2 ml/kg. Puis le nombre de protrusion de la langue et de mouvements de mastication est compté pendant 2 périodes de 5 minutes chacune à t=1 heure et à t=3 heures après administration du composé selon l'invention.

### Résultats :

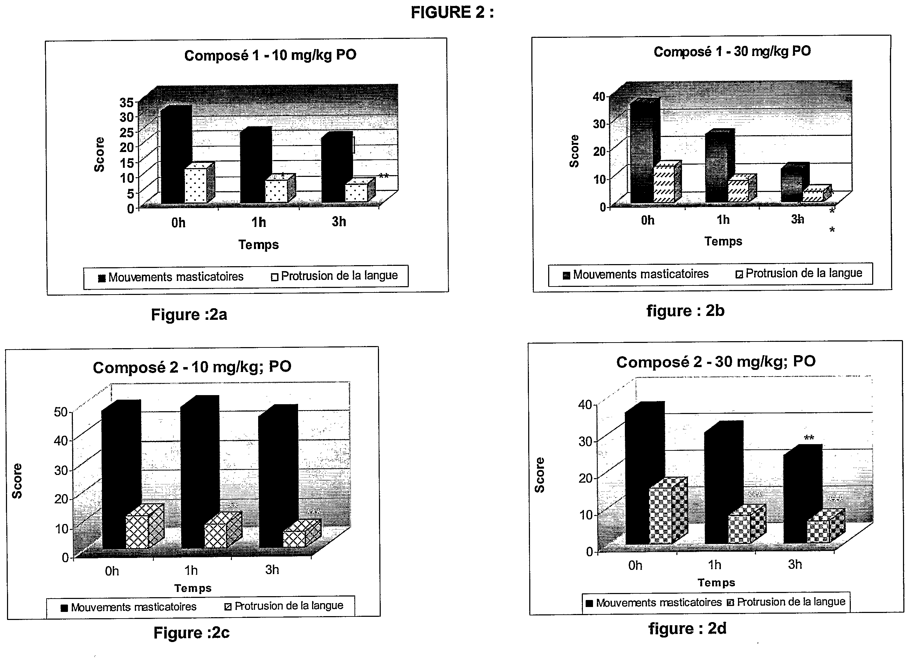

Le taux de protrusion de la langue et de mouvements de mastication est calculé de manière statistique pour chaque groupe de rats. Les résultats sont présentés sur la figure 2 a et b pour le composé 1 et sur la figure 2c et d pour le composé 2.

Les composés 1 et 2 selon l'invention sont administrés oralement à la dose de 10 et 30 mg/kg à des rats dyskinétiques selon le protocole de l'exemple 2 décrit ci-dessus. Les résultats présentés ont été obtenus avec 8 animaux testés.

Le score obtenu permet d'évaluer la dyskinésie du rat. Plus la valeur numérique du score est élevé, plus l'animal est dyskinétique.
- composé 1 : à la dose de 10 mg/kg, le score de mouvements masticatoires diminue de 30,3 à 21. A la dose de 30 mg/kg, le score de mouvements masticatoires diminue de 35,6 à 11,9.
- Composé 2 : à la dose de 10 mg/kg, le taux de protrusion de la langue a significativement diminué au bout de 3 heures, passant de 11,4 à 5. A la dose de 30 mg/kg, le nombre de mouvements de mastication et le taux de protrusion de la langue a significativement diminué au bout de 3 heures, passant de 35,5 à 23,7 et de 15 à 6 respectivement.

Les résultats indiquent une efficacité dose dépendante du traitement avec les composés 1 et 2 sur les rats atteints de dyskinésie tardive, dyskinésie orofaciale tardive provoquée par l'halopéridol. Les composés 1 et 2 ont bien un effet anti-dyskinétique.

## Revendications

1. Utilisation d'un composé de formule générale **(I)** 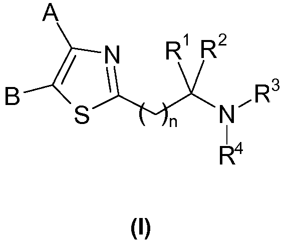 sous forme de mélange racémique, d'énantiomère ou de toute combinaison de ces formes, dans laquelle :
A représente un radical **(A1)** 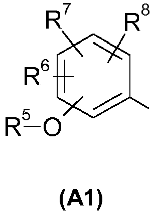
dans lequel R⁵ représente un atome d'hydrogène, R⁶, R⁷, R⁸ représentent indépendamment un atome d'hydrogène ou un radical alkyle;
B représente un atome d'hydrogène ou un radical alkyle ;
n représente 0 ;
R¹ représente un atome d'hydrogène et R² représente un atome d'hydrogène ou un radical alkyle;
R³ et R⁴ représentent indépendamment un atome d'hydrogène ou un radical alkyle,;
ou d'un sel de formule générale **(I)** définie ci-dessus pour préparer un médicament destiné à traiter ou à prévenir les dyskinésies provoquées par un traitement chimique.

2. Utilisation selon la revendication 1 **caractérisée en ce que** l'on utilise un composé de formule générale **(I)** ou un sel de formule générale **(I)** définie selon l'une des revendications précédentes pour préparer un médicament destiné à traiter ou à prévenir les dyskinésies provoquées par un traitement chimique comme par exemple la dyskinésie induite ou la dyskinésie tardive.

3. Utilisation selon l'une des revendications 1 à 2 **caractérisée en ce que** l'on utilise un composé de formule générale **(I)** ou un sel de formule générale **(I)** définie selon l'une des revendications précédentes pour préparer un médicament destiné à traiter ou à prévenir les dyskinésies induites par la lévodopa (L-Dopa), par l'assocation lévodopa-benzerazide ou l'association levodopa-carbodopa, par le pergolide, le quinpirole, la carbergoline, la benzotropine, le trihexylphénidyl, le ropinorole, le pramipexole et tout autres dérivés dopaminergiques se substituant à la dopamine.

4. Utilisation selon les revendications 1, 2 et 3 **caractérisée en ce que** l'on utilise un composé choisi parmi les composés suivants :
- 4-[2-(aminométhyl)-1,3-thiazol-4-yl]-2,6-di(*tert*-butyl)phénol ;
- 4-{2-[(1R)-1-aminoethyl]-1,3-thiazol-4-yl}-2,6-di-tert-butylphenol ;
- 4-{2-[(1S)-1-aminoethyl]-1,3-thiazol-4-yl}-2,6-di-tert-butylphenol.

5. Association d'un composé de formule générale **(I)** ou un sel de formule générale **(I)** définie selon la revendication 1 et d'au moins un composé choisi parmi les agonistes dopaminergiques, les inhibiteurs de MAO, les inhibiteurs de catécholamine O-methyltransférase ou les neuroleptiques.

6. Association selon la revendication 5 **caractérisée en ce qu'**elle associe un composé de formule générale **(I)** définie selon la revendication 1 ou son sel et de la L-Dopa.

7. Association selon l'une des revendications 5 ou 6 **caractérisée en ce qu'**elle associe le 4-[2-(aminométhyl)-1,3-thiazol-4-yl]-2,6-di(*tert*-butyl)phénol ou son sel et la L-Dopa.

8. Association selon l'une des revendications 5 ou 6 **caractérisée en ce qu'**elle associe le 4-{2-[(1R)-1-aminoethyl]-1,3-thiazol-4-yl}-2,6-di-tert-butylphenol ou son sel et la L-Dopa.

9. Association selon l'une des revendications 5 ou 6 **caractérisée en ce qu'**elle associe le 4-{2-[(1S)-1-aminoethyl]-1,3-thiazol-4-yl}-2,6-di-tert-butylphenol ou son sel et la L-Dopa.

10. Association selon l'une des revendications 5 à 9 comme préparation combinée pour une utilisation simultanée, séparée ou étalée dans le temps pour traiter ou prévenir les dyskinésies provoquées par un traitement chimique.

## Patentansprüche

1. Verwendung einer Verbindung der allgemeinen Formel (I) 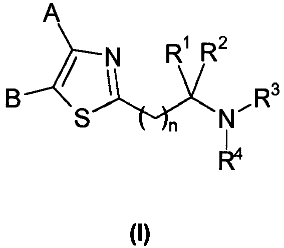 in Form eines racemischen Gemisches, in Enantiomerform oder allen Kombinationen dieser Formen, in der:
A einen Rest (A1) darstellt 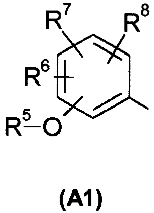
worin R⁵ ein Wasserstoffatom darstellt, R⁶, R⁷, R⁸ unabhängig ein Wasserstoffatom oder einen Alkylrest darstellen;
B ein Wasserstoffatom oder einen Alkylrest darstellt;
n 0 darstellt;
R¹ ein Wasserstoffatom darstellt und R² ein Wasserstoffatom oder einen Alkylrest darstellt;
R³ und R⁴ unabhängig ein Wasserstoffatom oder einen Alkylrest darstellen;
oder eines Salzes der allgemeinen Formel (I), die oben definiert ist, zur Herstellung eines Medikaments, das dazu bestimmt ist, Dyskinesien, die durch eine chemische Behandlung hervorgerufen werden, zu behandeln.

2. Verwendung nach Anspruch 1, **dadurch gekennzeichnet, dass** man eine Verbindung der allgemeinen Formel (I) oder ein Salz der allgemeinen Formel (I), definiert nach einem der vorangehenden Ansprüche, zur Herstellung eines Medikaments verwendet, das dazu bestimmt ist, Dyskinesien, die durch eine chemische Behandlung hervorgerufen werden, zum Beispiel induzierte Dyskinesie oder tardive Dyskinesie, zu behandeln oder zu verhindern.

3. Verwendung nach einem der Ansprüche 1 bis 2, **dadurch gekennzeichnet, dass** man eine Verbindung der allgemeinen Formel (I) oder ein Salz der allgemeinen Formel (I), definiert nach einem der vorangehenden Ansprüche, zur Herstellung eines Medikaments verwendet, das dazu bestimmt ist, Dyskinesien, die durch Levodopa (L-Dopa), durch die Kombination Levodopa-Benzerazid oder die Kombination Levodopa-Carbodopa, durch Pergolid, Quinpirol, Carbergolin, Benzotropin, Trihexylphenidyl, Ropinorol, Pramipexol und jegliche andere dopaminerge Derivate, die Dopamin ersetzen, induziert werden, zu behandeln oder zu verhindern.

4. Verwendung nach einem der Ansprüche 1, 2 und 3, **dadurch gekennzeichnet, dass** man eine Verbindung verwendet, die aus den folgenden Verbindungen ausgewählt ist:
- 4-[2-(Aminomethyl)-1,3-thiazol-4-yl]-2,6-di(tert-butyl)phenol;
- 4-{2-[(1R)-1-Aminoethyl]-1,3-thiazol-4-yl}-2,6-di-tert-butylphenol;
- 4-{2-[(1S)-1-Aminoethyl]-1,3-thiazol-4-yl}-2,6-di-tert-butylphenol.

5. Kombination aus einer Verbindung der allgemeinen Formel (I) oder einem Salz der allgemeinen Formel (I), definiert wie in Anspruch 1, und wenigstens einer Verbindung, die aus den dopaminergen Agonisten, den MAO-Inhibitoren, den Inhibitoren der Catecholamin-O-Methyltransferase und den Neuroleptika ausgewählt ist.

6. Kombination nach Anspruch 5, **dadurch gekennzeichnet, dass** sie eine Verbindung der allgemeinen Formel (I), definiert wie in Anspruch 1, oder ihr Salz und L-Dopa kombiniert.

7. Kombination nach einem der Ansprüche 5 oder 6, **dadurch gekennzeichnet, dass** sie 4-[2-(Aminomethyl)-1,3-thiazol-4-yl]-2,6-di(tert-butyl)phenol oder sein Salz und L-Dopa kombiniert.

8. Kombination nach einem der Ansprüche 5 oder 6, **dadurch gekennzeichnet, dass** sie 4-{2-[(1R)-1-Aminoethyl]-1,3-thiazol-4-yl}-2,6-di-tert-butylphenol oder sein Salz und L-Dopa kombiniert.

9. Kombination nach einem der Ansprüche 5 oder 6, **dadurch gekennzeichnet, dass** sie 4-{2-[(1S)-1-Aminoethyl]-1,3-thiazol-4-yl}-2,6-di-tert-butylphenol oder sein Salz und L-Dopa kombiniert.

10. Kombination nach einem der Ansprüche 5 bis 9 als Kombinationspräparat für eine gleichzeitige, getrennte oder zeitlich ausgedehnte Verwendung, um Dyskinesien, die durch eine chemische Behandlung hervorgerufen werden, zu behandeln oder zu verhindern.

## Claims

1. Use of a compound with the general formula **(I)** 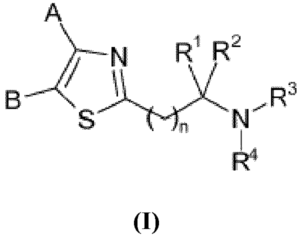 in the form of racemic mixture, an enantiomer or any combination of these forms, wherein:
A represents a radical **(A1)** 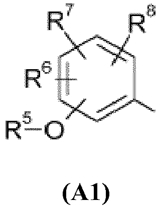
in which R⁵ represents a hydrogen atom and R⁶, R⁷ and R⁸ independently represent a hydrogen atom or an alkyl radical;
B represents a hydrogen atom or an alkyl radical;
n represents 0;
R¹ represents a hydrogen atom and R² represents a hydrogen atom or an alkyl radical;
R³ and R⁴ independently represent a hydrogen atom or an alkyl radical;
or of a salt with the general formula **(I)** defined above to prepare a drug for treating or preventing dyskinesias caused by a chemical treatment.

2. Use according to claim 1 **characterised in that** a compound with the general formula **(I)** or a salt with the general formula **(I)** defined according to one of the above claims is used to prepare a drug for treating or preventing dyskinesias caused by a chemical treatment such as for example induced dyskinesia or tardive dyskinesia.

3. Use according to one of claims 1 to 2 **characterised in that** a compound with the general formula **(I)** or a salt with the general formula **(I)** defined according to one of the above claims is used to prepare a drug for treating or preventing dyskinesias induced by levodopa (L-Dopa), by the levodopa-benserazide combination or the levodopa-carbodopa combination, by pergolide, quinpirole, cabergoline, benzotropine, trihexylphenidyl, ropinorole or pramipexole or by any other dopaminergic derivatives replacing dopamine.

4. Use according to claims 1, 2 and 3 **characterised in that** a compound chosen from the following compounds is used:
- 4-[2-(aminomethyl)-1,3-thiazol-4-yl]-2,6-di(*tert*-butyl)phenol;
- 4-{2-[(1R)-1-aminoethyl]-1,3-thiazol-4-yl}-2,6-di-tert-butylphenol;
- 4-{2-[(1S)-1-aminoethyl]-1,3-thiazol-4-yl}-2,6-di-tert-butylphenol.

5. Combination of a compound with the general formula **(I)** or a salt with the general formula **(I)** defined according to claim 1 and at least one compound chosen from dopaminergic agonists, MAO inhibitors, catecholamine O-methyltransferase inhibitors or neuroleptics.

6. Combination according to claim 5 **characterised in that** it combines a compound with the general formula **(I)** defined according to claim 1 or its salt and L-Dopa.

7. Combination according to one of claims 5 or 6 **characterised in that** it combines 4-[2-(aminomethyl)-1,3-thiazol-4-yl]-2,6-di(*tert*-butyl)phenol or its salt and L-Dopa.

8. Combination according to one of claims 5 or 6 **characterised in that** it combines 4-{2-[(1R)-1-aminoethyl]-1,3-thiazol-4-yl}-2,6-di-tert-butylphenol or its salt and L-Dopa.

9. Combination according to one of claims 5 or 6 **characterised in that** it combines 4-{2-[(1S)-1-aminoethyl]-1,3-thiazol-4-yl}-2,6-di-tert-butylphenol or its salt and L-Dopa.

10. Combination according to one of claims 5 to 9 as a combined preparation for simultaneous, separate or phased use to treat or prevent dyskinesias caused by a chemical treatment.
